(19) Europäisches Patentamt
European Patent Office
Office européen des brevets

(11) Veröffentlichungsnummer: **0 280 781 B1**

# (12) EUROPÄISCHE PATENTSCHRIFT

(45) Veröffentlichungstag der Patentschrift: **18.09.91**

(51) Int. Cl.$^5$: **C07D 233/32**, C07D 239/10

(21) Anmeldenummer: **87118461.0**

(22) Anmeldetag: **12.12.87**

## (54) Verfahren zur Herstellung von N-Alkyl-N'-methyl-alkylenharnstoffen, insbesondere N,N'-Dimethylalkylenharnstoffen.

(30) Priorität: **05.02.87 DE 3703389**

(43) Veröffentlichungstag der Anmeldung:
**07.09.88 Patentblatt 88/36**

(45) Bekanntmachung des Hinweises auf die Patenterteilung:
**18.09.91 Patentblatt 91/38**

(84) Benannte Vertragsstaaten:
**AT CH DE FR GB IT LI NL**

(56) Entgegenhaltungen:

**Keine Entgegenhaltungen**

(73) Patentinhaber: **HÜLS AKTIENGESELLSCHAFT**
**- RSP Patente / PB 15 - Postfach 13 20**
**W-4370 Marl 1(DE)**

(72) Erfinder: **Bickert, Peter, Dr.**
**318 Westgate Dr.**
**North Edison New Jersey, 08820(US)**
Erfinder: **Bellut, Hans, Dr.**
**Bergstrasse 50**
**W-4408 Dülmen(DE)**

EP 0 280 781 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstelllung von N-Alkyl-N'-methyl-alkylenharnstoffen bzw. N,N'-Dimethylalkylenharnstoffen aus den entsprechenden N-Alkyl-N'- (hydroxymethyl)-alkylenharnstoffen bzw. N,N'-Bis-(hydroxymethyl)-alkylenharnstoffen durch Umsetzung mit Ameisensäure, wobei "Alkylen" einen bifunktionellen organischen Rest bezeichnet, beispielsweise 1,2-Ethylen- oder 1,3-Propylen-. Vorzugsweise handelt es sich um die Herstellung von N,N'-Dimethylalkylenharnstoffen.

Die Umsetzung kann auch ohne Isolierung des N-Alkyl-N'-(hydroxymethyl)-alkylenharnstoffs bzw. des N,N'-Bis-(hydroxymethyl)-alkylenharnstoffs aus den entsprechenden Alkylenharnstoffen und Formaldehyd bzw. direkt aus Harnstoff und dem entsprechenden Diamin erfolgen.

Zur Herstellung von N,N'-Dialkylalkylenharnstoffen z. B. der nachstehenden Formel

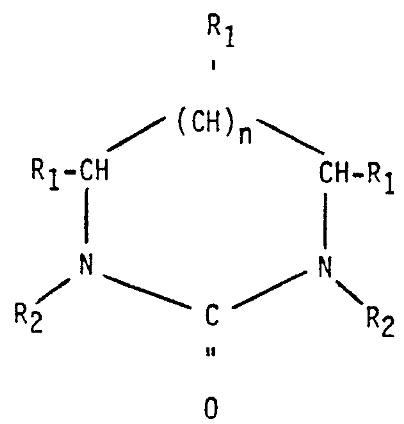

$R_1$ = H oder $CH_3$
$R_2$ = Alkyl mit 1 bis 3 C
n = 0 - 1
sind Verfahren bekannt, nach welchen man ein Alkylendiol mit einem Alkylamin und Kohlendioxid bei 150 bis 500 °C zur Reaktion bringt (JP 8150-565-A), die entsprechend alkylierten Alkylendiamine mit Phosgen umsetzt (DE-AS 11 26 392), die jeweiligen Alkylenthioharnstoffe alkyliert und hydrolisiert (J. med. Chem. 11, 214 (1968)) oder funktionalisierte Vorstufen mit $R_1$ = Alkyloxy reduziert (DE-OS 15 45 613).

Praktikable Verfahren zur Überführung von Alkylenharnstoffen in die entsprechenden N,N'-Dialkylalkylenharnstoffe sind allerdings nicht bekannt. Da jedoch Alkylenharnstoffe, z. B. Ethylenharnstoff, im technischen Maßstab verfügbar sind (US-PS 2 497 309) und sich daraus auch leicht N-Alkyl-N'-(hydroxymethyl)-alkylenharnstoffe bzw. N,N'-Bis-(hydroxymethyl)-alkylenharnstoffe bereiten lassen (US-PS 2 613 210), bestand die Aufgabe, diese in N-Alkyl-N'-methyl-alkylenharnstoffe bzw. N,N'-Dimethylalkylenharnstoffe umzuwandeln.

Diese Aufgabe wird entsprechend den Patentansprüchen gelöst. Gegenstand der Erfindung ist somit ein Verfahren zur Herstellung von N-Alkyl-N'-methyl-alkylenharnstoffen, insbesondere N,N'-Dimethylalkylenharnstoffen der Formel

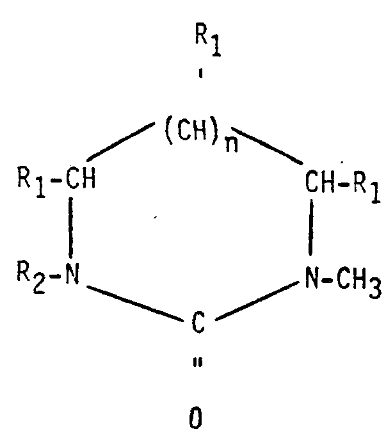

worin
$R_1$ = H oder $CH_3$ und
$R_2$ = $CH_3$, $C_2H_5$, $C_3H_7$, $C_4H_9$
n = 0 oder 1
aus den entsprechenden Alkylenharnstoffen und deren Umsetzung mit Formaldehyd zu den N-Alkyl-N'-(hydroxymethyl)-alkylenharnstoffen,insbesondere N,N'-Di(hydroxymethyl)-alkylenharnstoffen, dadurch gekennzeichnet, daß die N-Alkyl-N'-(hydroxymethyl)-alkylenharnstoffe bzw. N,N'-(Dihydroxymethyl)-alkylenharnstoffe mit Ameisensäure zur Reaktion gebracht werden.

Ein bewährtes Verfahren zur reduktiven Aminierung von Aldehyden und Ketonen stellt die als "Leuckart-Wallach-Reaktion" bekannte Umsetzung von Carbonylverbindungen mit Aminen in Gegenwart von Ameisensäure dar (Houben-Weyl XI/1, S. 648 ff). Die Eschweiler-Clark-Variante dieser Reaktion hat dabei als Methode zur Methylierung von Aminen mittels Formaldehyd und Ameisensäure besondere Bedeutung erlangt (Org. React. 5, 301 (1949)). Auf diese Weise wird beispielsweise Ethylendiamin in hervorragender Ausbeute in Tetramethylethylendiamin überführt. An gleicher Stelle (S. 308), wird jedoch ausdrücklich darauf hingewiesen, daß die Reaktion versagt, wenn polare Gruppen am Stickstoff gebunden sind, wie dies u. a. in Harnstoff der Fall ist. Solche Verbindungen sollen ausschließlich die Hydroxymethyl-Derivate ergeben.

Es ist weiterhin bekannt, daß Hydroxymethylalkylenharnstoffe wie N,N'-Bis-(hydroxymethyl)-ethylenharnstoff (Formel A, $R_1$ = H, $R_2$ = $CH_2OH$, n = 0) oder N,N'-Bis-(hydroxymethyl)-1,2-propylenharnstoff (Formel A, R = $CH_3$, $R_2$ = $CH_2OH$, n = 0) in Gegenwart von Ameisensäure unter Abspaltung von Wasser zu höhermolekularen Produkten reagieren, welche zur Behandlung von Cellulose eingesetzt werden können (US-PS 2 613 210).

Überraschend wurde nunmehr gefunden, daß N-Alkyl-N'-(hydroxymethyl)-alkylenharnstoffe, insbesondere N,N'-Bis-(hydroxymethyl)-alkylenharnstoffe durch Ameisensäure doch in die entsprechenden N-Alkyl-N'-methyl-alkylenharnstoffe bzw. N,N'-Dimethylalkylenharnstoffe überführt werden können. Dabei wirkt die Ameisensäure als Reduktionsmittel, das im Verlauf der Reaktion in Kohlendioxid umgewandelt wird.

Zur Herstellung von z. B. N,N'-Dimethylalkylenharnstoffen werden demnach ein aus Alkylenharnstoff und Formaldehyd bereiteter N,N'-Bis-(hydroxymethyl)-alkylenharnstoff, der gegebenenfalls nicht isoliert werden muß, mit einer wenigstens zweifach molaren und bis zehnfach molaren Menge Ameisensäure auf 50 bis 150 °C erhitzt. Bevorzugt ist ein Molverhältnis von Ameisensäure zu Bis-(hydroxymethyl)-alkylenharnstoff von 4 : 1 bis 7 : 1. Die Reaktion setzt bereits bei 50 °C ein, jedoch ist es vorteilhaft, das Gemisch zum Sieden zu erhitzen, welches bei Normaldruck etwa bei 100 bis 110 °C erfolgt. Alternativ dazu kann auch in geschlossenem System unter Eigendruck bei höherer Temperatur gearbeitet werden.

Nachdem die Reaktion beendet ist, je nach Temperatur nach etwa 6 bis 16 h, destilliert man überschüssige Ameisensäure bei Normaldruck oder leicht vermindertem Druck aus dem Reaktionsgemisch ab. Dabei verbleibt jedoch Ameisensäure im Reaktionsgefäß, die sich vom gebildeten N-Alkyl-N'-methyl-alkylenharnstoff bzw. N,N'-Dimethylalkylenharnstoff nicht vollständig abtrennen läßt.

Zur Bereitung eines ameisensäurefreien N-Alkyl-N'-methyl-alkylenharnstoffes bzw. N,N'-Dimethylalkylenharnstoffes behandelt man das Rohprodukt entweder mit einer Base, beispielsweise einem Alkali- oder Erdalkalihydroxid oder -carbonat und destilliert ein Produkt hoher Reinheit ab, oder man setzt einen niederen Alkohol und katalytische Mengen einer Mineralsäure zu, welche die Ameisensäure in einen Ester überführt, der destillativ abgetrennt werden kann.

Eine vorteilhafte Variante dieses Verfahrens stellt dabei der Verzicht auf eine destillative Gewinnung des rohen N-Alkyl-N'-methyl-alkylenharnstoffes bzw. N,N'-Dimethylalkylenharnstoffes aus dem primär anfallenden Stoffgemisch dar. So ist es möglich, bereits im Reaktionsgefäß durch Zugabe der Base oder Umsetzung mit einem Alkohol und nachfolgende Destillation den gewünschten Dialkylalkylenharnstoff direkt zu gewinnen. Dadurch gelingt es, eine extraktive Aufarbeitung durch Extraktion des Reaktionsproduktes mittels Chloroform aus wäßriger Lösung zu vermeiden, wie sie bei der Herstellung von Dialkylalkylenharnstoffen durch Alkylierung und Hydrolyse von Alkylenthioharnstoffen (J. med. Chem. 11, 214 (1968)) erforderlich ist.

Eine Steigerung der Ausbeute des erfindungsgemäßen Verfahrens ist möglich, wenn man der eingesetzten Ameisensäure zur Abstumpfung des Säurecharakters eine gewisse Menge einer Base bereits zu Beginn der Reaktion zusetzt. Hierzu ist Natriumformiat besonders geeignet. Der Zusatz an Formiat kann, bezogen auf eingesetzte Ameisensäure, bei 1 Gew.-% bis zur Sättigungskonzentration, insbesondere 5 bis 30 Gew.-%, liegen. Als optimale Konzentration hat sich eine Einsatzmenge von 10 bis 15 Gew.-% Natriumformiat bezüglich der eingesetzten Menge Ameisensäure erwiesen.

N-Alkyl-N'-methyl-alkylenharnstoffe bzw. N,N'-Dimethylalkylenharnstoffe, insbesondere N,N'-Dimethylethylenharnstoff (1,3-Dimethylimidazolidin-2-on) und N,N'-Dimethyl-1,3-propylenharnstoff (1,3-Dimethyltetrahydro-2(1H)-pyrimidinon), besitzen Bedeutung als polare, aprotische Lösungsmittel, die u. a. als geeigneter Ersatz für das cancerogene Hexamethylphosphorsäuretriamid empfohlen werden (Nachr. Chem. Technik u. Labor 33, 396 (1985); Chemistry in Britain 1985, 632).

## Beispiele

1 a) Propylenharnstoff (Tetrahydro-2(1H)-pyrimidinon)

126 g (2,10 mol) Harnstoff, 148 g (2,0 mol) Propylendiamin, 120 g (1,94 mol) Ethylenglykol und 3 g (0,08 mol) NaOH werden unter Rühren und Durchleiten eines langsamen Stickstoffstromes erhitzt. Dabei soll die Temperatur der Mischung kontinuierlich von 115 °C auf 160 °C in 2 Stunden steigen. Man läßt weitere 2 Stunden nachreagieren und nutscht nach Abkühlen ab. Nach Waschen mit Aceton wird im Vakuum getrocknet.
Das zurückgewonnene Ethylenglykol kann für den nächsten Ansatz wieder verwendet werden.

Ausbeute 184 g Propylenharnstoff, entsprechend 92 % mit Fp = 250 bis 60 °C

| Analyse: | C | H | N | |
|---|---|---|---|---|
| | 48,0 | 8,1 | 28,3 | gef. |
| | 48,0 | 8,0 | 28,0 | ber. |

1 b) Dimethylpropylenharnstoff (1,3-Dimethyl-tetrahydro-2(1H)-pyrimidinon)

40 g (0,4 mol) Propylenharnstoff und 92 g (2,0 mol) Ameisensäure (100 %) mit Wasser auf 85 % verdünnt werden bei 0 °C vorgelegt. Dazu werden 28,8 g (0,96 mol) Formaldehyd als 36 %ige wäßrige Formalinlösung getropft. In exothermer Reaktion beginnt die $CO_2$-Abspaltung. Nach 1 Stunde bei Raumtemperatur wird für 24 Stunden auf 100 bis 105 °Cgeheizt. Nach Eindampfen alkalisiert man mit methanolischer NaOH, nutscht ab und destilliert im Feinvakuum $Kp_2$ = 81 bis 83 °C.

Ausbeute 43,2 g Dimethylpropylenharnstoff, entsprechend 84,7 % mit $n_D^{20}$ = 1,4888 sowie GC 99,2 %

| Analyse: | C | H | N | O | $H_2O$ | |
|---|---|---|---|---|---|---|
| | 55,0 | 9,3 | 21,7 | 13,4 | 0,78 | gef. |
| | 56,3 | 9,4 | 21,9 | 12,5 | - | ber. |

2 a) Ethylenharnstoff (Imidazolidin-2-on)

Analog 1 a) werden 63 g (1,05 mol) Harnstoff, 61,5 g (1,02 mol) Ethylendiamin, 84 g (1,35 mol) Ethylenglykol und 1,5 g (0,04 mol) NaOH bis zur Endtemperatur von 200 °C umgesetzt und aufgearbeitet. Da Ethylenharnstoff eine merkliche Löslichkeit in Glykol besitzt, werden gute Ausbeuten nur bei Rückführung des Glykols erzielt. Dies ist auch bei dem hier beschriebenen Versuch der Fall.

Ausbeute 75,2 g Ethylenharnstoff, entsprechend 87,4 % mit Fp = 130 °C

| Analyse: | C | H | N | O | |
|---|---|---|---|---|---|
| | 41,7 | 7,1 | 32,6 | 19,0 | gef. |
| | 41,8 | 7,0 | 32,6 | 18,6 | ber. |

2 b) Dimethylethylenharnstoff (1,3-Dimethylimidazolidin-2-on)

Analog 1 b) werden 43 g (0,5 mol) Ethylenharnstoff mit 135 g (2,35 mol) Ameisensäure 85 %ig und 90 ml (1,11 mol) Formalin 37 %ig umgesetzt und aufgearbeitet. $Kp_3$ = 70 bis 2 °C.
Ausbeute 44,5 g Diemthylethylenharnstoff, entsprechend 78 % mit $n_D^{20}$ = 1,4724 sowie GC 99,8 %.

| Analyse: | C | H | N | O | $H_2O$ | |
|---|---|---|---|---|---|---|
| | 51,0 | 8,9 | 24,4 | 16,0 | 0,3 | gef. |
| | 52,6 | 8,8 | 24,6 | 14,0 | - | ber. |

Beispiel 3

64 g (0,44 mol) 1,3-Bis(hydroxymethyl)imidazolidin-2-on wurden mit 124 g (2,7 mol) Ameisensäure 8 h zum Rückfluß erhitzt. Danach destillierte man 86 g Ameisensäure unter vermindertem Druck ab. Der Rückstand wurde in 150 ml 20 %iger Natronlauge aufgenommen und dreimal mit je 50 ml Chloroform extrahiert. Die vereinigten organischen Phasen trocknete man mit Natriumsulfat und engte ein. Destillation des Rückstandes im Vakuum ergab bei 85 bis 90 °C/2 mbar 28 g (56 %) 1,3-Dimethylimidazolidin-2-on einer Reinheit von > 99 % gemäß GC und $^1$H-NMR.

Beispiel 4

320 g (2,2 mol) 1,3-Bis(hydroxymethyl)imidazolidin-2-on und 200 g (2,9 mol) Natriumformiat wurden in 620 g (13,5 mol) Ameisensäure gelöst und 8 h zum Rückfluß erhitzt. Danach destillierte man 238 g Ameisensäure unter vermindertem Druck ab und versetzt den Rückstand portionsweise mit 200 g Kaliumcarbonat. Nach dem Abkühlen dekantierte man das Rohprodukt von den ausgeschiedenen Salzen ab, wusch mit wenig Methylenchlorid nach und destillierte im Vakuum. Bei 90 bis 95 °C/3 mbar erhielt man 157 g (63 %) 1,3-Dimethylimidazolidin-2-on einer Reinheit von > 98 % gemäß GC und $^1$H-NMR.

Beispiel 5

147 g (1,0 mol) 1,3-Bis(hydroxymethyl)imidazolidin-2-on und 9 g (0,1 mol) Natriumformiat wurden in 95 g (2,0 mol) Ameisensäure 8 h zum Rückfluß erhitzt. Nach destillativer Abtrennung der Hauptmenge nicht umgesetzter Ameisensäure erhielt man bei 70 bis 72 °C/1 mbar 23 g eines ameisensäurehaltigen Rohproduktes. Dieses wurde mit überschüssigem Kaliumcarbonat versetzt. Die anschließende Vakuum-Destillation lieferte 16 g (14 %) 1,3-Dimethylimidazolidin-2-on einer Reinheit von > 98 % gemäß GC und $^1$H-NMR.

Beispiel 6

147 g (1,0 mol) 1,3-Bis(hydroxymethyl)imidazolidin-2-on und 54 g (0,8 mol) Natriumformiat wurden mit 570 g (12,4 mol) Ameisensäure 4 h unter Rückfluß erhitzt. Danach destillierte man 425 g Ameisensäure unter vermindertem Druck ab und versetzte den Rückstand mit 800 g Methanol und 48 g konz. Schwefelsäure. Dieses Gemisch erhitzte man 3 h zum Rückfluß, filtrierte vom gebildeten Natriumsulfat ab und engte

ein. Die Destillation des Rückstandes im Vakuum ergab bei 90 bis 95 °C/3 mbar 80 g (70 %) 1,3-Dimethylimidazolidin-2-on in Reinheit von > 98 % gemäß GC und $^1$H-NMR.

Beispiel 7

Der als Beispiel 6 beschriebene Ansatz wurde mit verlängerten Reaktionszeiten unter sonst gleichen Bedingungen wiederholt. Bei einer Reaktionszeit von 8 h wurde danach 97 g (85 %), nach 16 h wurden 98 g (86 %) 1,3-Dimethylimidazolidin-2-on erhalten.

Beispiel 8

Eine Mischung aus 29,4 g (0,20 mol) 1,3-Bis(hydroxymethyl)-imidazolidin-2-on, 115,0 g (2,5 mol) Ameisensäure und 10,2 g (0,15 mol) Natriumformiat wurden im Autoklav 1,5 h auf 150 °C erhitzt. Nach Aufarbeitung mit 200 ml Methanol und 9 g Schwefelsäure wie in Beispiel 6 beschrieben erhielt man 16,4 g (72 %) 1,3-Dimethylimidazolidin-2-on (> 98 % gemäß GC und $^1$H-NMR).

Beispiel 9

Unter den Bedingungen und dem Stoffeinsatz nach Beispiel 8 wurden mit Reaktionszeiten von 4 bzw. 8 h Ausbeuten von 18,0 g (79 %) bzw. 16,2 g (71 %) erhalten.

Beispiel 10

80 g (0,5 mol) 1,3-Bis(hydroxymethyl)tetrahydro-2(1H)-pyrimidinon und 27 g (0,4 mol) Natriumformiat wurden mit 272 g (5,9 mol) Ameisensäure 8 h unter Rückfluß erhitzt. Danach destillierte man 194 g Ameisensäure unter vermindertem Druck ab und versetzte den Rückstand mit 400 ml Methanol und 17 g konz. Schwefelsäure. Dieses Gemisch erhitzte man 3 h zum Rückfluß, saugte vom gebildeten Natriumsulfat ab und engte ein. Die Destillation des Rückstandes im Vakuum ergab bei 85 bis 90 °C/1 mbar 51 g (80 %) 1,3-Dimethyltetrahydro-2(1H)-pyrimidinon in einer Reinheit von > 98 % gemäß GC und $^1$H-NMR.

Beispiel 11

Nach dem in Beispiel 10 angegebenen Verfahren erhielt man aus 80 g (0,5 mol) 1,3-Bis-(hydroxymethyl)-4-methylimidazolidin-2-on 47 g (71 %) 1,3,4-Trimethylimidazolidin-2-on vom Sdp. 62 bis 64/10,5 mbar (> 98 %) gemäß GC und $^1$H-NMR.

Beispiel 12

Nach dem in Beispiel 10 angegebenen Verfahren erhielt man aus 103 g (0,5 mol) 1,3-Bis-(hydroxymethyl)-4,4,6-trimethyltetrahydro-2(1H)-pyrimidinon 66 g (76 %) 1,3,4,4,6-Pentamethyltetrahydro-2-(1H)-pyrimidinon vom Sdp. 97 bis 101/0,4 mbar, Schmp. 36,5 °C (> 98 % gemäß GC und $^1$H-NMR).

Beispiel 13

72 g (0,5 mol) 1-Ethyl-3-hydroxymethylimidazolidin-2-on und 14 g (0,2 mol) Natriumformiat wurden mit 138 g (6,0 mol) Ameisensäure 8 h unter Rückfluß erhitzt. Danach destillierte man 95 g Ameisensäure unter vermindertem Druck ab und versetzte den Rückstand mit 200 ml Methanol sowie 8 g konz. Schwefelsäure. Dieses Gemisch erhitzte man 3 h zum Rückfluß, saugte vom gebildeten Natriumsulfat ab und engte ein. Die Destillation des Rückstandes im Vakuum ergab bei 80 bis 84 °C/0,8 mbar 46 g (72 %) 1-Ethyl-3-methylimidazolidin-2-on (> 98 % gemäß GC und $^1$H-NMR).

Beispiel 14

Nach dem in Beispiel 13 angegebenen Verfahren erhielt man aus

a) 79 g (0,5 mol) 1-n-Propyl-3-hydroxymethylimidazolidin-2-on 48 g (68 %) 1-Methyl-3-n-propylimidazolidin-2-on (Sdp. 88 bis 91 °C/1,0 mbar);

b) 79 g (0,5 mol) 1-i-Propyl-3-hydroxymethylimidazolidin-2-on 43 g (61 %) 1-Methyl-3-i-propylimidazolidin-2-on (Sdp. 83 bis 87 °C/0,9 mbar);

c) 86 g (0,5 mol) 1-t-Butyl-3-hydroxymethylimidazolidin-2-on 33 g (42 %) 1-t-Butyl-3-methylimidazolidin-2-on (Sdp. 96 bis 100 °C/1,2 mbar), sowie

d) 93 g (0,5 mol) 1-t-Butyl-3-hydroxymethyltetrahydro-2(1H)-pyrimidinon38 g (45 %) 1-t-Butyl-3-methyltetrahydro-2(1H)-pyrimidinon (Sdp. 100 bis 103 °C/1,1 mbar)

entsprechender Reinheit.

## Patentansprüche

1. Verfahren zur Herstellung von N-Alkyl-N'-methyl-alkylenharnstoffen, insbesondere N,N'-Dimethylalkylenharnstoffen der Formel

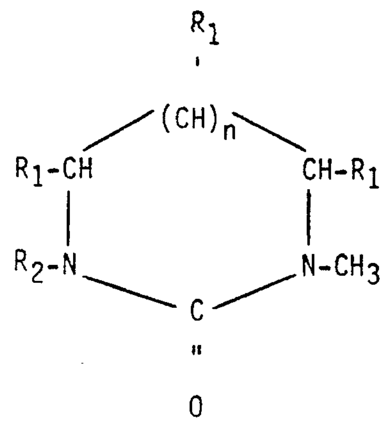

worin
$R_1$ = H oder $CH_3$ und
$R_2$ = $CH_3$, $C_2H_5$, $C_3H_7$, $C_4H_9$
n = 0 oder 1
aus den entsprechenden Alkylenharnstoffen und deren Umsetzung mit Formaldehyd zu den N-Alkyl-N'-(hydroxymethyl)-alkylenharnstoffen,insbesondere N,N'-Di(hydroxymethyl)-alkylenharnstoffen,
dadurch gekennzeichnet,
daß die N-Alkyl-N'-(hydroxymethyl)-alkylenharnstoffe bzw. N,N'-(Dihydroxymethyl)-alkylenharnstoffe mit Ameisensäure im Molverhältnis 1:2 bis 1:10 zur Reaktion gebracht werden.

2. Verfahren nach Anspruch 1,
dadurch gekennzeichnet,
daß man N-Alkyl-N'-(hydroxymethyl)-alkylenharnstoff bzw. N,N'-Bis(hydroxymethyl)-alkylenharnstoff mit Ameisensäure bei erhöhtem Druck oder - bevorzugt - bei Normaldruck, zur Reaktion bringt.

3. Verfahren nach den Ansprüchen 1 und 2,
dadurch gekennzeichnet,
daß man N-Alkyl-N'-(hydroxymethyl)-alkylenharnstoff bzw. N,N'-Bis(hydroxymethyl)-alkylenharnstoff mit Ameisensäure im Temperaturbereich 50 bis 150 °C, bevorzugt bei Rückflußtemperatur des Reaktionsgemisches bei Normaldruck, entsprechend 100 bis 110 °C, zur Reaktion bringt.

4. Verfahren nach den Ansprüchen 1 bis 3,
dadurch gekennzeichnet,
daß man die Ameisensäure im Molverhältnis 1 : 4 bis 1 : 7, bezogen auf N-Alkyl-N'-(hydroxymethyl)-alkylenharnstoff bzw. N,N'-Bis(hydroxymethyl)-alkylenharnstoff einsetzt.

5. Verfahren nach den Ansprüchen 1 bis 4,
dadurch gekennzeichnet,
daß man dem Reaktionsgemisch aus N-Alkyl-N'-(hydroxymethyl)-alkylenharnstoff bzw. N,N'-Bis-(hydroxymethyl)-alkylenharnstoff und Ameisensäure eine Base, bevorzugt ein Alkali- oder Erdalkalisalz einer Carbonsäure, besonders bevorzugt Natriumformiat, zusetzt.

6. Verfahren nach Anspruch 5,

dadurch gekennezeichnet,
daß man dem Reaktionsgemisch die Base in einer Konzentration von 1 Gew.-%, bezogen auf eingesetzte Ameisensäure, bis zur Sättigungskonzentration, bevorzugt 5 bis 30 Gew.-%, besonders bevorzugt 10 bis 15 Gew.-%, zusetzt.

**7.** Verfahren nach den Ansprüchen 1 bis 6,
dadurch gekennzeichnet,
daß man den aus Alkylenharnstoff und Formaldehyd in wäßriger Lösung anfallenden N-Alkyl-N′-(hydroxymethyl)-alkylenharnstoff bzw. N,N′-Bis(hydroxymethyl)-alkylenharnstoff direkt ohne vorherige Aufarbeitung einsetzt.

**8.** Verfahren nach den Ansprüchen 1 bis 7,
dadurch gekennzeichnet,
daß man die im rohen N-Alkyl-N′-methylalkylenharnstoff bzw. N,N′-Dimethylalkylenharnstoff verbliebene Ameisensäure durch Zuatz von Alkali- oder Erdalkalihydroxid oder -carbonat entfernt.

**9.** Verfahren nach den Ansprüchen 1 bis 7,
dadurch gekennzeichnet,
daß man die im rohen N-Alkyl-N′-methylalkylenharnstoff bzw. N,N′-Dimethylalkylenharnstoff verbliebene Ameisensäure mit einem niedrigsiedenden Alkohol, bevorzugt Methanol, umsetzt und in Gegenwart einer Mineralsäure in den gebildeten Ameisensäureester überführt, welchen man destillativ abtrennt.

**10.** Verfahren nach einem der Ansprüche 1 bis 9,
dadurch gekennzeichnet,
daß man den N-Alkyl, N′-methylalkylenharnstoff bzw. N,N′-Dimethylalkylenharnstoff durch Destillation aus dem Reaktionsgemisch gewinnt.

**Claims**

**1.** A process for the preparation of N-alkyl-N'-methyl-alkylene ureas, more particularly N,N'-dimethyl alkylene ureas having the Formula

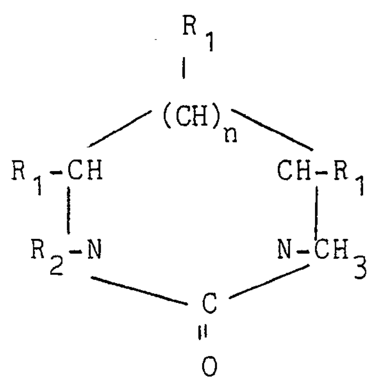

where
$R_1$ = H or $CH_3$ and
$R_2$ = $CH_3$, $C_2H_5$, $C_3H_7$, $C_4H_9$
n = 0 or 1
from the corresponding alkylene ureas and their reaction with formaldehyde to give the N-alkyl-N'-(hydroxymethyl)-alkylene ureas, more particularly N,N'-di(hydroxymethyl)-alkylene ureas, characterized in that the N-alkyl-N'-(hydroxymethyl)-alkylene ureas or the N,N'-(dihydroxymethyl)-alkylene ureas are reacted with formic acid in the molar ratio 1 : 2 to 1 : 10.

**2.** A process according to claim 1, characterized in that N-alkyl-N'-(hydroxymethyl)-alkylene urea or N,N'-bis-(hydroxymethyl)-alkylene urea are reacted with formic acid at elevated pressure or preferably at normal pressure.

3. A process according to claims 1 and 2, characterized in that N-alkyl-N'-(hydroxymethyl)-alkylene urea or N,N'-bis-(hydroxymethyl)-alkylene urea is reacted with formic acid in the temperature range 50 to 150 °C, preferably at the reflux temperature of the reaction mixture and at normal pressure, corresponding to 100 to 110 °C.

4. A process according to claims 1 to 3, characterized in that the formic acid is used in a molar ratio of 1 : 4 to 1 : 7, referred to N-alkyl-N'-(hydroxymethyl)-alkylene urea or N,N'-bis(hydroxymethyl)-alkylene urea.

5. A process according to claims 1 to 4, characterized in that a base, preferably an alkaline or alkaline earth salt of a carboxylic acid, particularly preferably sodium formate, is added to the reaction mixture of N-alkyl-N'-(hydroxymethyl)alkylene urea or N,N'-bis-(hydroxymethyl)-alkylene urea and formic acid.

6. A process according to claim 5, characterized in that the base is added to the reaction mixture in a concentration of 1% by weight, referred to the formic acid used, up to the saturation concentration, preferably 5 to 30% by weight, particularly preferably 10 to 15% by weight.

7. A process according to claims 1 to 6, characterized in that the N-alkyl-N'-(hydroxymethyl)-alkylene urea or N,N'-bis-(hydroxymethyl)-alkylene urea obtained in aqueous solution from alkylene urea and formaldehyde is used directly without preceding processing.

8. A process according to claims 1 to 7, characterized in that the formic acid remaining in the crude N-alkyl-N'-methyl alkylene urea or N,N'-dimethyl alkylene urea is removed by the addition of alkaline or alkaline earth hydroxide or carbonate.

9. A process according to claims 1 to 7, characterized in that the formic acid remaining in the crude N-alkyl-N'-methyl alkylene urea or N,N'-dimethyl alkylene urea is reacted with a low-boiling alcohol, preferably methanol, and converted in the presence of a mineral acid into the formic acid ester formed, which is separated by distillation.

10. A process according to one of claims 1 to 9, characterized in that the N-alkyl-N'-methyl alkylene urea or N,N'-dimethyl alkylene urea is obtained from the reaction mixture by distillation.

**Revendications**

1. Procédé d'obtention de N-alcoyl-N'-méthylalcoylène urée, en particulier de N,N'-diméthylalcoylène urée de formule :

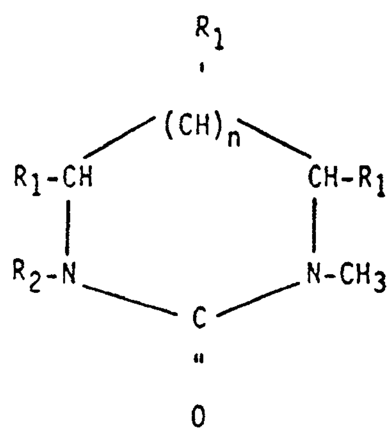

dans laquelle :
$R_1$ = H ou $CH_3$
$R_2$ = $CH_3$, $C_2H_5$, $C_3H_7$ ou $C_4H_9$
n = 0 ou 1

à partir des alcoylène urée correspondantes et de leur réaction avec le formaldéhyde en N-alcoyl-N'-(hydroxyméthyl)alcoylène urée, en particulier en N,N'-di(hydroxyméthyl)alcoylène urée, caractérisé en ce que les N-alcoyl-N'-(hydroxyméthyl)alcoylène urée ou les N,N'-(dihydroxyméthyl)alcoylène urée sont mises en réaction avec l'acide formique dans un rapport molaire de 1 : 2 à 1 : 10.

**2.** Procédé selon la revendication 1, caractérisé en ce que l'on met en réaction la N-alcoyl-N'-(hydroxyméthyl)alcoylène urée ou la N,N'-bis-(hydroxyméthyl)alcoylène urée avec de l'acide formique à pression élevée ou - de préférence à pression ordinaire.

**3.** Procédé selon les revendications 1 et 2, caractérisé en ce que l'on met en réaction la N-alcoyl-N'-(hydroxyméthyl)alcoylène urée ou la N,N'-bis-(hydroxyméthyl)alcoylène urée avec de l'acide formique dans la zone de température allant de 50 à 150°C, de préférence à la température de reflux du mélange réactionnel à pression ordinaire, ce qui correspond à 100°-110°C.

**4.** Procédé selon les revendications 1 à 3, caractérisé en ce que l'on met en oeuvre l'acide formique dans un rapport molaire allant de 1 : 4 à 1 : 7, rapporté à la N-alcoyl-N'(hydroxyméthyl)alcoylène urée ou à la N,N'-bis-(hydroxyméthyl)alcoylène urée.

**5.** Procédé selon les revendications 1 à 4, caractérisé en ce que l'on ajoute au mélange réactionnel à base de N-alcoyl-N'-(hydroxyméthyl)-alcoylène urée ou de N,N'-bis-(hydroxyméthyl)alcoylène urée et d'acide formique, une base, de préférence un sel de métal alcalin ou alcalino-terreux d'un acide carboxylique d'une manière particulièrement préférée le formiate de sodium.

**6.** Procédé selon la revendication 5, caractérisé en ce que l'on ajoute au mélange réactionnel une base à une concentration à partir de 1 % en poids, rapporté à l'acide formique mis en oeuvre - jusqu'à la concentration de saturation, de préférence de 5 à 30 % en poids, et d'une manière particulièrement préférée de 10 à 15 % en poids.

**7.** Procédé selon les revendications 1 à 6, caractérisé en ce que l'on met en oeuvre la N-alcoyl-N'-(hydroxyméthyl)alcoylène urée ou la N,N'-bis-(hydroxyméthyl)alcoylène urée qui s'est formée en solution aqueuse, à partir d'alcoylène urée et de formaldéhyde, directement sans purification préalable.

**8.** Procédé selon les revendications 1 à 7, caractérisé en ce que l'on élimine l'acide formique qui est demeuré dans la N-alcoyl-N'-méthylalcoylène urée ou la N,N'-diméthylalcoylène urée brutes, par addition d'un hydroxyde ou d'un carbonate de métal alcalin ou de métal alcalino-terreux.

**9.** Procédé selon les revendications 1 à 7, caractérisé en ce que l'on fait réagir l'acide formique qui demeure dans la N-alcoyl-N'-méthylalcoylène urée ou la N,N'-diméthylalcoylène urée avec un alcool à bas point d'ébullition, de préférence du méthanol, que l'on transforme en présence d'un acide minéral en un ester d'acide formique formé qu'on élimine par distillation.

**10.** Procédé selon l'une des revendications 1 à 9, caractérisé en ce que l'on récupère la N-alcoyl-N'-méthylalcoylène urée ou la N,N'-diméthylalcoylène urée, par distillation, à partir du mélange réactionnel.